# EUROPEAN PATENT SPECIFICATION

(11) **EP 4 442 312 B1**
(45) Date of publication and mention of the grant of the patent: **10.12.2025**
(21) Application number: 22901590.4
(22) Date of filing: 02.11.2022
(51) Int. Cl.: A61N 1/40, A61N 1/06, A61N 1/08, A61N 1/32, A61N 1/04, A61N 1/36, A61B 18/00, A61B 18/14

(54) **ANTI-BURN RF ELECTRODE CARTRIDGE FOR SKIN CARE AND TREATMENT, AND HANDPIECE FOR SKIN CARE AND TREATMENT COMPRISING SAME**
RF-ELEKTRODENKARTUSCHE MIT BRENNSCHUTZ ZUR HAUTPFLEGE UND -BEHANDLUNG SOWIE HANDSTÜCK ZUR HAUTPFLEGE UND -BEHANDLUNG DAMIT
CARTOUCHE D'ÉLECTRODE RF ANTI-BRÛLURE POUR SOINS ET TRAITEMENT DE LA PEAU, ET PIÈCE À MAIN POUR SOINS ET TRAITEMENT DE LA PEAU LA COMPRENANT

(30) Priority: 02.12.2021 KR 20210170714
(43) Date of publication of application: 09.10.2024
(73) Proprietor: CLASSYS INC., Seoul 06220 (KR)
(72) Inventor: PARK, Si Hyung, Seoul 08329 (KR); LEE, Seok Joo, Seoul 01781 (KR); JEONG, Ha Gil, Seoul 08735 (KR)
(74) Representative: Zimmermann, Tankred Klaus
(86) International application number: PCT/KR2022/016970
(87) International publication number: WO 2023/101231

(56) References cited:
- KR-A- 20160 114 712
- KR-A- 20200 085 450
- KR-A- 20200 085 450
- KR-B1- 102 048 383
- KR-B1- 102 048 384
- KR-B1- 102 168 547
- US-A1- 2004 111 087
- US-A1- 2008 200 969
- US-A1- 2014 188 099

## Description

### [Technical Field]

The present disclosure relates to an anti-burn RF electrode cartridge for skin care and treatment, and a handpiece for skin care and treatment comprising the same. The present disclosure relates to an anti-burn RF electrode cartridge for skin care and treatment that keeps the part, where an RF electrode is concentrated, away from the skin during a procedure by positioning the edge end of the RF electrode, and a handpiece for skin care and treatment including the anti-burn RF electrode cartridge.

### [Background Art]

In general, a handpiece with an RF electrode is a device that treats the skin and the tissues under the skin by heating them using the RF electrode (radio frequency). KR 20200085450A shows an anti-burn RF electrode cartridge for skin care and treatment, the anti-burn RF electrode cartridge comprising a tip part on a bottom of which an RF electrode assembly configured to apply RF energy into skin is positioned; and a cartridge casing having an opening at a lower portion at which the tip part is positioned, wherein the RF electrode assembly positioned to cover the entire bottom of the tip part.

Handpieces with an RF electrode are used to restore the skin by increasing the temperature of the skin after cryo fat reduction for obesity treatment and are used for skin treatment that generates contraction of collagen or causes a wound healing response by heating the skin or the tissues under the skin.

Recently, as the interest in skin care increases, handpieces with an RF electrode are used in various ways such as remodeling/resurfacing of the skin by inducing contraction of collagen in the lower derma or a wound healing response, removing of wrinkles, and treatment of sebaceous glands, adipose tissues of hair follicles, and spider veins.

A handpiece with an RF electrode includes an RF electrode cartridge including an RF electrode assembly to come in contact with the skin of a patient, and a handpiece body casing to which the RF electrode cartridge is separably coupled and that is connected to a control body.

Handpieces with an RF electrode are implemented in various patterns to fit to the types of skin treatment by controlling an electrical signal that is applied to an RF electrode on a surface of an RF electrode cartridge, thereby increasing the effect of corresponding skin treatment.

Accordingly, a relay for controlling an electrical signal that is applied to an RF electrode is disposed at a handpiece body casing.

An RF electrode cartridge is an expendable of which the number of times is set in advance, and is replaced when the preset available number of times is reached.

Further, since the relay disposed at a handpiece body casing is also an expendable of which the number of times is set in advance, when a handpiece body casing is also replaced when the number of times of the relay is reached.

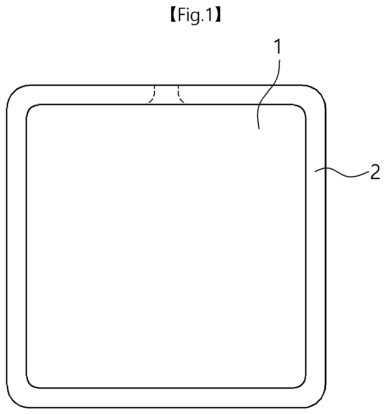

Meanwhile, FIG. 1 is a bottom view showing an RF electrode cartridge for skin care and treatment of the related art. Referring to FIG. 1, according to RF electrode cartridge for skin care and treatment of the related art, an RF electrode is positioned on the bottom of a tip supposed to come in contact with the skin and an RF electrode is positioned inside the bottom of the tip.

That is, referring to FIG. 1, an RF electrode 1 is mounted such that the edge thereof is positioned inside the outer periphery of the bottom of a tip 2.

The RF electrode 1 has a characteristic that when an RF signal is applied, RF energy concentrates to the edge of the RF electrode, that is, the outer periphery of the RF electrode 1.

Accordingly, when an RF signal is generated through the RF electrode 1 with the tip 2 of the RF electrode cartridge for skin care and treatment in contact with the skin, the patient may get burned by the RF energy concentrating to the edge of the RF electrode, that is, the outer periphery of the RF electrode 1 during a procedure.

In particular, when the RF electrode 1 is in contact with the skin during a procedure, the edge of the RF electrode 1, that is, the part where the RF energy concentrates is spaced apart from the skin due to a curved surface of the skin, that is, the elasticity of the skin in many cases, and, in this case, there is a problem that the patient gets burned due to a spark.

As a prior patent relating to the present disclosure, there is a 'Fluid RF Electrode Assembly for Beauty Treatment of Skin and Handpiece for Beauty Treatment of Skin using The Same' in Korean Patent No. 2048384 (registered on 2019.11.19).

### [Disclosure]

### [Technical Problem]

An objective of the present disclosure is to provide an RF electrode cartridge for skin care and treatment that keeps the part, where an RF electrode is concentrated, away from the skin during a procedure by positioning the edge end of the RF electrode, and a handpiece for skin care and treatment including the RF electrode cartridge.

Another objective of the present disclosure is to provide an RF electrode cartridge for skin care and treatment that can minimize pain, which is generated during skin treatment, and can improve a treatment effect by having a vibration motor, and a handpiece for skin care and treatment including the RF electrode cartridge.

Another objective of the present disclosure is to provide an RF electrode cartridge for skin care and treatment that enables safe and convenient treatment because electrical power is applied to an RF electrode assembly only when the RF electrode cartridge is pressed in contact state with the skin, and a handpiece for skin care and treatment including the RF electrode cartridge.

### [Technical Solution]

In order to achieve the objectives of the present disclosure described above, an embodiment of an anti-burn RF electrode cartridge for skin care and treatment according to the present disclosure includes: a tip part on a bottom of which an RF electrode assembly configured to apply RF energy into the skin is positioned; and a cartridge casing having an opening at a lower portion at which the tip part is positioned, wherein the RF electrode assembly positioned to cover the entire bottom of the tip part and extend to an outer surface of the tip part, thereby surrounding the outer surface of the tip part.

In order to achieve the objectives of the present disclosure described above, a handpiece for skin care and treatment according to the present disclosure includes: an RF electrode cartridge comprising an RF electrode assembly supposed to come in contact with the skin; and a handpiece body casing to which the RF electrode cartridge is separably coupled and that is connected to a control body, wherein the RF electrode cartridge is an embodiment of the anti-burn RF electrode cartridge for skin care and treatment according to the present disclosure.

### [Advantageous Effects]

Since the edge end of an RF electrode that is the part where RF energy concentrates during a procedure is positioned on a side of a tip, he present disclosure prevents an accident of generation of a spark between the edge end of the RF electrode and the skin during a procedure, thereby having an effect of preventing a patient from getting a burn on the skin during skin treatment that uses an RF signal and greatly improving safety during a procedure.

The present disclosure includes a vibration motor, thereby having an effect of minimizing pain that is generated during skin treatment and improving treatment effect.

Since electrical power is applied to an RF electrode assembly only when an RF electrode cartridge that is in contact with the skin is pressed, the present disclosure has an effect of being able to perform safe and simple treatment and improving safety in treatment.

### [Description of Drawings]

FIG. 1 is a bottom view showing an RF electrode cartridge for skin care and treatment of the related art.
FIG. 2 is a perspective view showing an embodiment of a handpiece for skin care and treatment according to the present disclosure.
FIG. 3 is an exploded perspective view showing an embodiment of an anti-burn RF electrode cartridge for skin care and treatment according to the present disclosure.
FIG. 4 is a cross-sectional view showing an embodiment of an anti-burn RF electrode cartridge for skin care and treatment according to the present disclosure.
FIG. 5 is an enlarged cross-sectional view showing a portion of the anti-burn RF electrode cartridge for skin care and treatment according to the present disclosure.
FIG. 6 is an exploded perspective view showing an embodiment of a handpiece for skin care and treatment according to the present disclosure.
FIG. 7 is a cross-sectional view showing the embodiment of a handpiece for skin care and treatment according to the present disclosure.
FIG. 8 is a plan view of an RF electrode cartridge for skin care and treatment in an embodiment of a handpiece for skin care and treatment according to the present disclosure.
FIG. 9 is a bottom view of a handpiece body casing in an embodiment of a handpiece for skin care and treatment according to the present disclosure.

***Description of reference numerals***

| | | | |
|---|---|---|---|
| 100 : | RF electrode cartridge | 101 : | tip part |
| 110 : | cartridge casing | 110a : | first casing part |
| 110b : | second casing part | 110c : | opening |
| 111 : | cooling space | 112 : | temperature sensor |
| 113 : | terminal insulating part | 114 : | cooling space cover part |
| 114a : | ball coupling part | 115 : | electrode on-off switch |
| 115a : | switch button | 115b : | switch spring |
| 116 : | switch-mounting protrusion | 120 : | RF electrode assembly |
| 121 : | contact electrode part | 121a : | electrode connection terminal |
| 122 : | side contact part | 123 : | insulating film member |
| 130 : | cartridge lock | | |
| 140 : | ball joint part | | |
| 150 : | bellows member | 150a : | pin insertion part |
| 151 : | bellows member support framemember | 152 : | frame fixing pin |
| 160 : | vibration motor part | 170 : | first cooling water line part |
| 180 : | second cooling water line part | 192 : | first body-connecting terminal part |
| 200 : | handpiece body casing part | 200a : | cooling water circulation line |
| 200b: | cartridge insertion part | 210 : | cartridge couplign block |
| 211 : | second body-connecting terminal part | 212 : | line connection part |
| 220 : | sparing member for cartridge | 230 : | cartridge movement guide part |
| 231 : | movement guide rod part | 232 : | movement guide block |
| 240 : | protrusion insertion part | 241 : | switch-pressing protrusion |

### [Mode for Invention]

The present disclosure is described in detail hereafter. The present invention is defined by the appended claims.

Exemplary embodiments of the present disclosure are described hereafter in detail with reference to the accompanying drawings. Before describing the present disclosure, it should be noted that the terms or terminologies used herein and claims should not be construed as common meanings or the meanings in dictionaries.

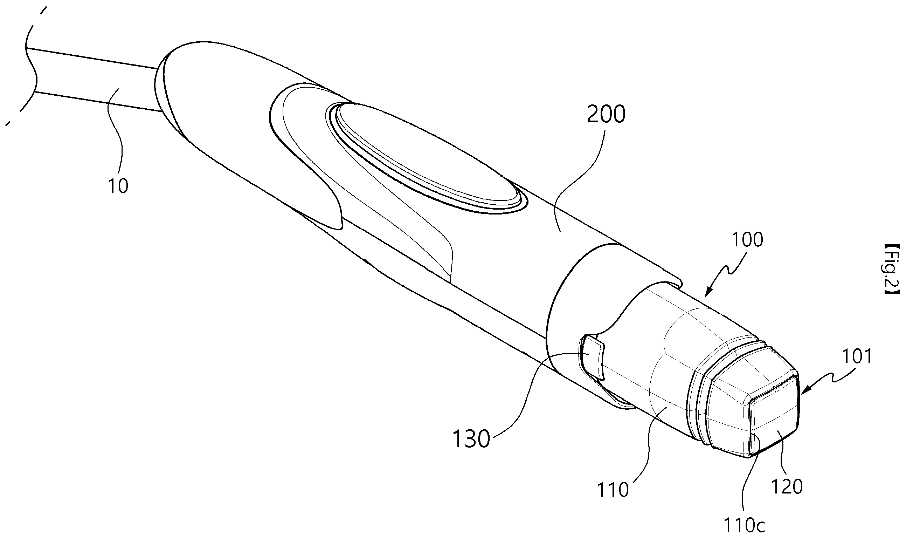

FIG. 2 is a perspective view showing an embodiment of a handpiece for skin care and treatment according to the present disclosure. Referring to FIG. 1, an embodiment of a handpiece for skin care and treatment according to the present disclosure includes an RF electrode cartridge 100 including an RF electrode assembly 120 supposed to come in contact with the skin, and a handpiece body casing 200 to which the RF electrode cartridge 100 is separably coupled and that is connected to a control body.

An embodiment of a handpiece for skin care and treatment including an anti-burn RF electrode cartridge 100 for skin care and treatment according to the present disclosure includes an RF electrode cartridge 100 including an RF electrode assembly 120 supposed to come in contact with the skin, and a handpiece body casing 200 to which the RF electrode cartridge 100 is separably coupled and that is connected to a control body.

The handpiece body casing 200 is connected to a control body through a connection cable 10, and the control body can be modified in various ways as a well-known component including an RF signal generator that generates an RF signal by applying AC power to the RF electrode assembly 120, so it should be noted that the control body is not further described in detail.

Meanwhile, according to the handpiece for skin care and treatment including an anti-burn RF electrode cartridge 100 for skin care and treatment according to the present disclosure, AC power is applied to the RF electrode assembly 120 from the control body with the handpiece held and the RF electrode assembly 120 in contact with the skin by a hand of a practitioner to generate an RF signal through the RF signal generator so that the skin is treated or a beauty effect can be obtained by heating the derma in the skin.

An RF electrode panel assembly (not shown) having a larger area than the RF electrode assembly 120 may be positioned opposite to the handpiece for skin care and treatment including the anti-burn RF electrode cartridge 100 according to the present disclosure.

The RF electrode panel assembly is a well-known component that can treat the skin or obtain a beauty effect by heating the derma in the skin by applying an RF signal, so it should be noted that the RF electrode panel assembly is not further described in detail.

That is, the handpiece for skin care and treatment according to the present disclosure heats the tissues in the skin by applying an RF signal to the skin with the RF electrode assembly 120 in contact with the skin, thereby being able to perform remodeling/resurfacing of the skin by inducing contraction of collagen in the lower derma or a wound healing response, removing of wrinkles, and treatment of sebaceous glands, adipose tissues of hair follicles, and spider veins.

The anti-burn RF electrode cartridge 100 is an expandable that cannot be used for skin treatment and is replaced because the lifespan ends when it is used for treatment for a preset time, and is separably coupled to the handpiece body casing 200.

A cartridge insertion part 200b in which a portion of the RF electrode cartridge 100 is inserted and coupled is positioned at the front end of the handpiece body casing 200.

The RF electrode cartridge 100 is inserted in the cartridge insertion part 200b, coupled to a cartridge coupling block 210 positioned inside the cartridge insertion part 200b, and locked by cartridge locks 130 at a final coupling position, whereby coupling is finished.

The cartridge locks 130 are button-type locks positioned on both sides of the cartridge casing 110 and use a well-known configuration that has elastically supported buttons having wedges, which lock and support the cartridge coupling block 210, and that is unlocked when the buttons are pressed, so it should be noted that the cartridge block 130 is not further described in detail.

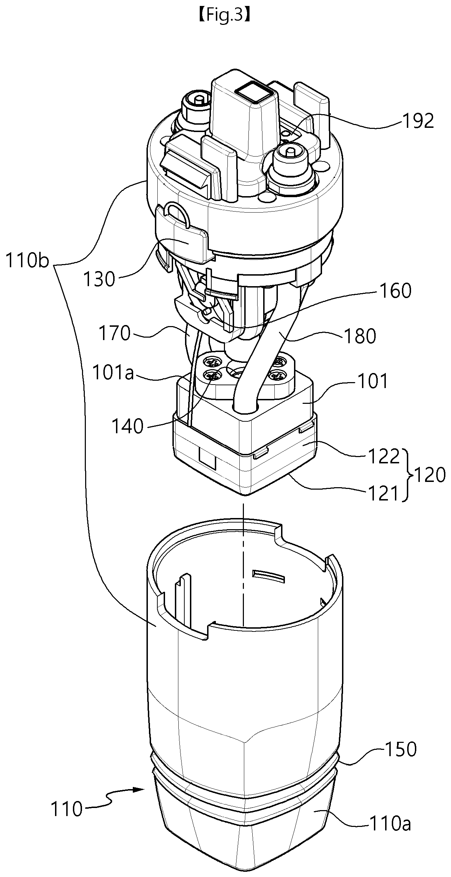

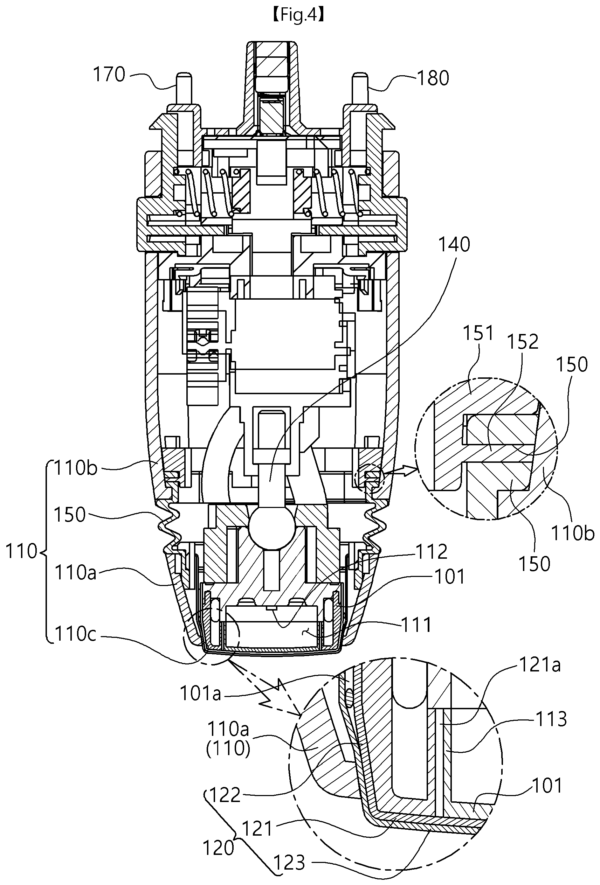

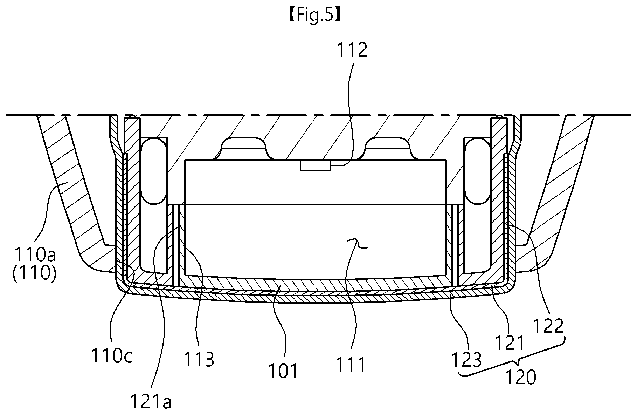

Meanwhile, FIG. 3 is an exploded perspective view showing an embodiment of an anti-burn RF electrode cartridge for skin care and treatment according to the present disclosure, FIG. 4 is a cross-sectional view showing an embodiment of an anti-burn RF electrode cartridge for skin care and treatment according to the present disclosure, and FIG. 5 is an enlarged cross-sectional view showing a portion of the anti-burn RF electrode cartridge for skin care and treatment according to the present disclosure.

An embodiment of an anti-burn RF electrode cartridge for skin care and treatment according to the present disclosure is described hereafter in detail with reference to FIG. 3 to FIG. 5.

An embodiment of an anti-burn RF electrode cartridge 100 for skin care and treatment according to the present disclosure includes a tip part 101 on the bottom of which an RF electrode assembly 120 applying RF energy into the skin is positioned, and a cartridge casing having an opening 110c at the lower portion of which the tip part 101 is positioned.

The RF electrode assembly 120 is positioned on the bottom of the tip part 101, the RF electrode assembly 120 is positioned to cover the entire bottom of the tip part 101 and extend to the outer surface of the tip part 101.

The RF electrode assembly 120 includes a contact electrode part 121 covering the entire bottom of the tip part 101 and a side electrode part 122 connected to the contact electrode part 121 and surrounding the side of the tip part 101.

The contact electrode part 121 and the side electrode part 122 have an integrally connected structure, and the end of the RF electrode assembly 120, that is, the edge of the RF electrode assembly 120 is positioned to face up on the outer surface of the tip part 101.

It is exemplified that the RF electrode assembly 120 is integrally formed by plating on the bottom and the outer surface of the tip part 101.

It is exemplified that the contact electrode part 121 covering the entire bottom of the tip part 101 and the side electrode part 122 connected to the contact electrode part 121 and surrounding the outer surface of the tip part 101 are plating layers integrally formed by plating.

The RF electrode assembly 120 is formed as a plating layer, so it covers the entire bottom of the tip part 101 and can be easily formed in a shape surrounding the outer surface.

The end of the RF electrode assembly 120, that is, the edge of the RF electrode assembly 120, at which RF energy concentrates during a procedure in which the RF electrode assembly 120 generates an RF signal, faces up on the side of the tip part 101, so it is possible to prevent an accident that a patient gets burned due to the RF energy concentrating at the edge of the RF electrode assembly 120, that is, the outer periphery of the RF electrode assembly 120 during a procedure while generating an RF signal through the RF electrode assembly 120.

That is, the contact electrode part 121 of the RF electrode assembly 120 that is in contact with the skin during a procedure covers the entire bottom of the tip part 101 and the end of the RF electrode assembly 120, to which an RF signal is applied, is positioned to face up on the outer surface of the tip part 101, is spaced apart from the skin of a patient, and is not contact with the skin of the patient by the side electrode part 122 integrally formed with the contact electrode part 121 to surround the outer surface of the tip part 101, whereby it is possible to completely prevent an accident that the patient gets burned due to a spark that is generated when the contact with the skin is removed.

Meanwhile, the RF electrode assembly 120 further includes an insulating film member 123 that covers the entire surface of the contact electrode part 121 and covers at least a portion of the side contact part 122.

The insulating film member 123 prevents the RF electrode assembly 120 from directly coming in contact with the skin of a patient by covering the surface of the contact electrode part 121 supposed to come in contact with the skin.

The RF electrode assembly 120 is connected to an RF signal application wire part 101a for applying AC power on the outer surface of the tip part 101, that is, on the side electrode part 122.

The RF electrode assembly 120 receives AC power through the RF signal application wire part 101a connected to the side electrode part 122, thereby stably generating an RF signal to the surface of the contact electrode part 121 supposed to come in contact with the skin.

Further, the RF electrode assembly 120 is formed to have a curved surface that is downwardly convex and to form a curved surface that is not deformed when it is pressed onto the skin.

It is exemplified that the surface curvature of the RF electrode assembly 120 has a curvature that can maximize the area that comes in contact with the skin when it is pressed on the skin.

Further, the RF electrode assembly 120 is formed in a shape in which the surface is a convex curved surface, that is, has a uniform curvature 360° around the center, so it has a shape that can uniformly distribute pressure when pressing the skin, and can prevent pain due to excessive pressing of the skin by the edges of the outer periphery.

The RF electrode assembly 120 is formed to make a curved surface that is not deformed when it is pressed on the skin, so it is possible to maximize the area that comes in close contact with the skin when pressing the skin and it is possible to secure durability because RF electrode assembly is not deformed in use.

The bottom of the tip part 101 is formed to have a curved surface being convex downward and to make a curved surface that is not deformed when it is pressed on the skin such that the RF electrode assembly 120 formed on the surface thereof by plating, that is, the contact electrode part 121 can be formed as a curved surface being convex downward.

Since the RF electrode assembly 120, that is, the contact electrode part 121 has a convex curved surface, it can be uniformly brought in close contact with the skin, which is a treatment part, even though it is pressed by a small force during a procedure, so it is possible to secure convenience for a practitioner and minimize inconvenience and pain of a patient during a procedure.

The cartridge casing 110 includes a first casing part 110a on a surface of which the RF electrode assembly 120 is positioned, and a second casing part 110b to which the first casing part 110a is connected to be able to tilt and has an opening 110c at the lower portion through which the tip part 101 passes, so the cartridge casing 110 can keep in contact with the skin by tilting when the RF electrode assembly 120 is moved in contact with the skin.

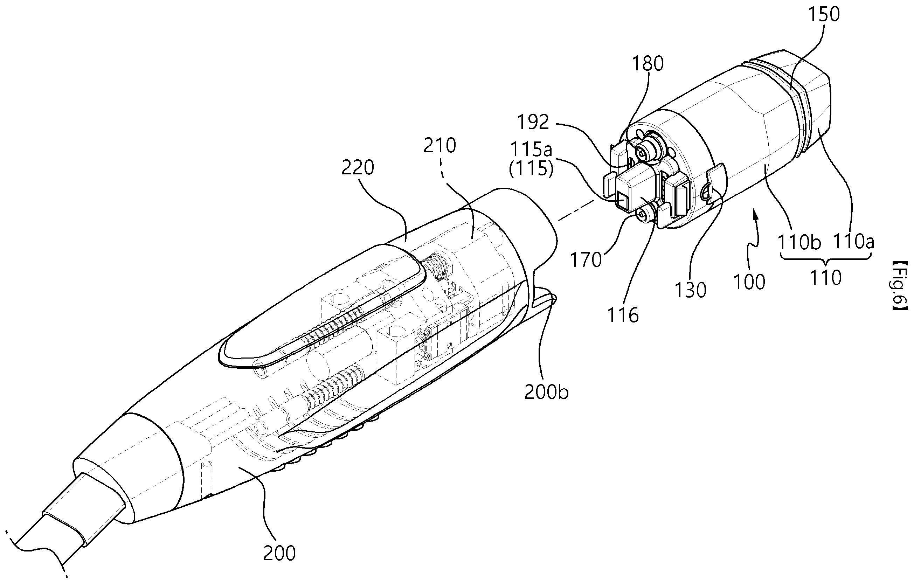

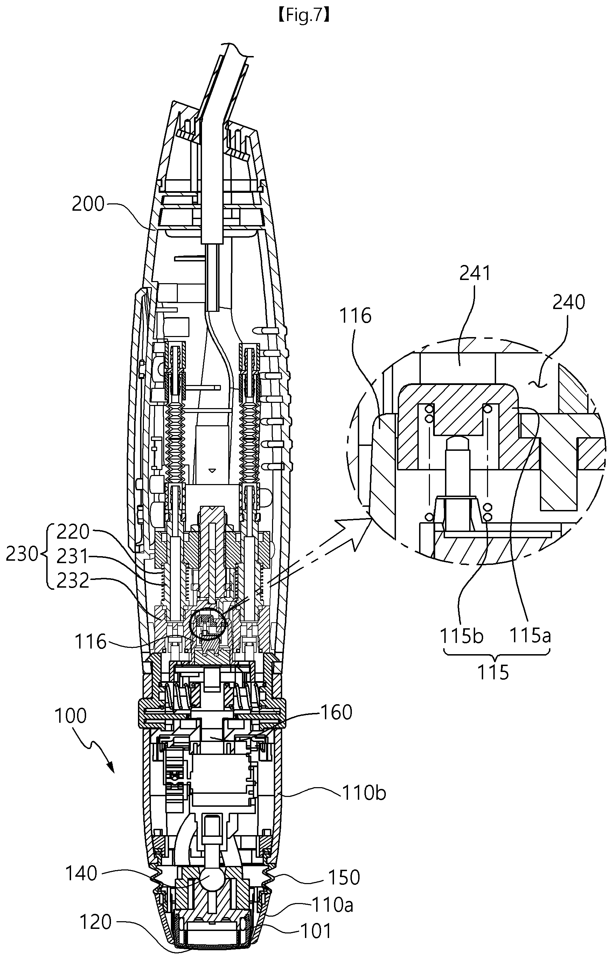

FIG. 6 is an exploded perspective view showing an embodiment of a handpiece for skin care and treatment according to the present disclosure, FIG. 7 is a cross-sectional view showing the embodiment of a handpiece for skin care and treatment according to the present disclosure, and an embodiment of the anti-burn RF electrode cartridge 100 for skin care and treatment according to the present disclosure is described hereafter in detail with reference to FIG. 6 to FIG. 7.

The second casing part 110b is a part that is separably coupled to the handpiece body casing 200. A casing coupling part that is inserted in the cartridge insertion part 200b of the handpiece body casing 200 and coupled with the cartridge coupling block 210 is positioned at a first end of the second casing part 110b.

The cartridge casing 110 has a first body-connecting terminal part 192 for electrically connecting the RF electrode assembly 120 to the control body.

It is exemplified that the first body-connecting terminal part 192 is positioned to be exposed on the top of the cartridge casing 110, that is, the top of the second casing part 110 to be described below.

It is exemplified that the first body-connecting terminal part 192 is electrically connected to the control body by being electrically connected to a second body-connecting terminal part 211 positioned in the cartridge insertion part 200b of the handpiece body casing 200 to be described below, and it should be noted that this will be described below in more detail in the description of an embodiment of a handpiece for skin care and treatment according to the present disclosure.

Further, the first casing part 110a is connected to be able to tilt to a second end of the second casing part 110b.

In more detail, the anti-burn RF electrode cartridge 100 for skin care and treatment according to the present disclosure further includes a ball joint part 140 connecting the second casing part 110b and the tip part 101 such that the tip part 101 and the first casing part 110a can freely tilt within 360 degrees.

It is exemplified that the ball joint part 140 includes a joint support rod member fixed to the second end of the second casing part 110b and a ball member positioned at an end of the joint support rod and rotatably coupled to the tip part 101.

A ball insertion part in which the ball member is inserted and rotatably positioned is positioned at the tip part 101, the tip part 101 can freely tilt within 360 degrees around the ball member, and the first casing part 110a through which the tip part 101 is positioned can also freely tilt within 360 degrees together with the tip part 101.

Further, an embodiment of the anti-burn RF electrode cartridge 100 for skin care and treatment according to the present disclosure may further include a bellows member 150 of which a first end is connected to the first casing part 110a and a second end is connected to the second casing part 110b and that is positioned to surround the ball joint part 140 and has a plurality of wrinkle parts.

The plurality of wrinkle parts of the bellows member 150 is positioned in the longitudinal direction of a handpiece for skin care and treatment, so the wrinkle parts support tilting operation of the first casing part 110a and the tip part 101 while freely folding and unfolding.

The first end of the bellows part 150 is fixed along the outer periphery of the first casing part 110a and the second end thereof is fixed along the outer periphery of the second casing part 110b, so the bellows part 150 surrounds the outer side of the ball joint part 140 positioned at the center at a spaced position.

The bellows member 150 enables the first casing part 110a and the tip part 101 to freely tilt around the ball member within 360 degrees by surrounding the outer side of the ball joint part 140 at a spaced position, and prevents foreign substances, etc. from entering the first casing part 110a and the second casing part 110b by covering the portion between the first casing part 110a and the second casing part 110b.

Further, an embodiment of the anti-burn RF electrode cartridge 100 for skin care and treatment according to the present disclosure further includes a bellows support frame member 151 positioned at the upper end of the bellows member 150 and formed in a frame structure surrounding the inner periphery of the bellows member 150.

It is exemplified that the bellows support frame member 151 has a frame structure forming a space therein, through which the ball joint part can pass, by surrounding the inner periphery of the bellows member 150 and has a rectangular frame structure, and it should be noted that the bellows support frame member 151 may be deformed in various frame shapes in accordance with the shape of the bellows member 150, that is, the shape of the connection portion of the first casing part 110a and the second casing part 110b.

Further, the bellows support frame member 151 has a plurality of frame fixing pins 150a protruding from the outer surface thereof, and the bellows member 150 has a plurality of pin insertion parts 150a in which the frame fixing pins 150a are inserted.

The frame fixing pins 150a protrude from the outer surface of the bellows support frame member 151 and are inserted and coupled in the pin insertion parts 150a of the bellows member 150, thereby stably fixing the position of the bellows support frame member 151.

The bellows support frame member 151 is positioned at the upper end of the bellows member 150 that is connected to the second casing part 110b, thereby being able to reinforce the strength at the connection portion of the bellows member 150 and prevent an accident of deformation or breakage of the bellows member 150 at the connection portion of the second casing part 110b and the bellows member 150.

Further, an embodiment of the anti-burn RF electrode cartridge 100 for skin care and treatment according to the present disclosure may further include a vibration motor part 160 positioned in the cartridge casing 110.

The vibration motor part 160 is positioned at a ball joint support part to which the ball joint part is connected in the second casing part 110b and generates vibration during a procedure, thereby being able to further improve the treatment effect through a massage effect on the skin on which the RF electrode assembly 120 is in close contact, and being able to reduce pain of a patient that is generated by electrical stimulation during treatment.

The tip part 101 has a cooling space 111 filled with cooling water for cooling the RF electrode assembly 120 and the anti-burn RF electrode cartridge 100 for skin care and treatment according to the present disclosure includes a first cooling water line part 170 and a second cooling water line part 180 for supplying cooling water into the cooling space 111 and discharging the cooling water in the cooling space 111 such that the cooling water circulates.

The cooling space 111 is positioned to include the bottom of the tip part 101 on which the RF electrode assembly 120 is positioned.

When the anti-burn RF electrode cartridge 100 for skin care and treatment according to the present disclosure is coupled to a handpiece for skin care and treatment, the first cooling water line part 170 and the second cooling water line part 180 are connected to a cooling water circulation line positioned in the handpiece for skin care and treatment.

The first cooling water line part 170 and the second cooling water line part 180 are positioned to protrude upward from the cartridge casing 110, thereby having a structure that is connected to a cooling water circulation line part 200a of the handpiece body casing 200 when the casing coupling part is inserted into the cartridge insertion part 200b and coupled to the cartridge coupling block 210.

The cooling water circulation line part 200a is connected to a cooling water tank positioned in the control body. The first cooling water line part 170 and the second cooling water line part 180 are connected to the cooling water tank positioned in the control body through the cooling water circulation line part 200a.

Cooling water passes through the cooling tank and the cooling space 111 through the cooling water circulation line part 200a, the first cooling water line part 170, and the second cooling water line part 180 and is then circulated by operation of a pump, and is cooled by a cooling part positioned in the cooling water circulation line or the cooling water tank during circulation, whereby the cooling water can be maintained at a predetermined temperature in the cooling space 111.

The cooling part may include a well-known cooler that cools cooling water such as a radiator or a chiller.

Further, a temperature sensor 112 that senses the temperature of cooling water is positioned in the cooling space 111, and senses and transmits the temperature of cooling water to a controller in the control body.

The controller controls operation of the pump and the cooling part through temperature information of cooling water received from the temperature sensor, thereby being able to maintain the temperature of the cooling water in the cooling space 111 at a temperature suitable for cooling the skin during treatment.

It is exemplified that the temperature sensor 112 is positioned in the cooling space 111, measures the temperature of cooling water at which the skin is substantially cooled, and maintains the temperature of cooling water at a temperature suitable for cooling the skin, and it should be noted that the temperature sensor 112 may be positioned at any positions where it can measure the temperature of cooling water, such as the cooling water circulation line or the cooling water tank.

Cooling water is cooled by the cooling pat through the cooling water tank after discharged from the cooling space 111 by operation of the pump, and then flows back into the cooling space 111, thereby being able to cool the surface of the skin when the skin is heated by application of an RF signal.

That is, it is possible to prevent the skin from getting burned or being injured by heat in treatment by cooling the surface of the skin, which is being treated, by lowering the temperature of the RF electrode assembly 120, that is, the contact electrode part 121 that is heated during a procedure, using the circulating cooling water.

The anti-burn RF electrode cartridge 100 for skin care and treatment according to the present disclosure can continuously and stably cool the skin can cool the skin at a uniform and constant temperature using circulating cooling water by circulating the cooling water in the cooling space 111, thereby being able to minimize unpleasantness due to cooling of skin and improve treatment satisfaction in skin treatment.

Meanwhile, the contact electrode part 121 may have an electrode connection terminal 121a protruding upward and electrically connected to the RF signal generator of the control body.

A plurality of electrode connection terminals 121a is positioned and the lower ends thereof are connected to the RF electrode assembly 120 through the cartridge casing 110.

Further, the electrode connection terminals 121a protrude upward from the cartridge casing 10 through the cooling space 111 and are electrically connected to the first body-connecting terminal part 192 positioned on the top of the cartridge casing 110, that is, the top of the second casing part 110b to be exposed, whereby they can be electrically connected to the control body.

The cooling space 111 has terminal passages through which the electrode connection terminals 121a pass, and has a terminal insulating part 113 insulating the electrode connection terminals 121a from the cooling water in the cooling space 111.

A cooling space cover part 111 having a ball coupling part 114a coupled with a ball joint is positioned on the upper portion of the cooling space 111, whereby the cooling space has a structure of which the inside is sealed.

The terminal insulating part 113 integrally protrudes from the bottom of the cooling space 111 and is connected to the cooling space cover part 111 sealing the upper portion of the cooling space with the upper end thereof, whereby the electrode connection terminals 121a can be positioned to be able to be electrically connected to the control body outside the cooling space 111 through the cooling space 111 with an insulation state from cooling water.

Since the RF electrode assembly 120 is integrally formed as a plating layer on the bottom and the outer surface of the tip part 101, the present disclosure can secure economic efficiency by simplifying the manufacturing process and reducing the manufacturing cost.

Further, since the RF electrode assembly 120 has a fixed shape that is not deformed even though it is pressed by the skin, the present disclosure secures durability of the RF electrode assembly 120.

Further, since cooling water is circulated, the present disclosure can maintain the surface temperature of the skin by cooling the surface of the skin that is heated by an RF signal, so it is possible to minimize unpleasantness due to cooling of the skin and improve treatment efficiency and treatment satisfaction in treatment.

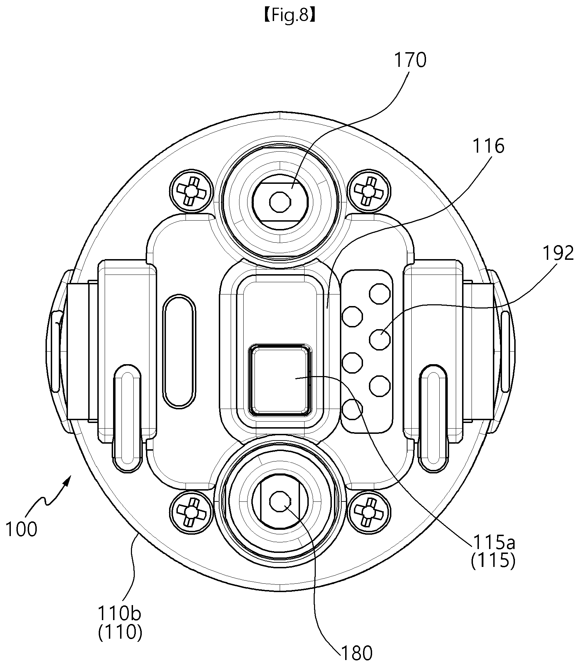

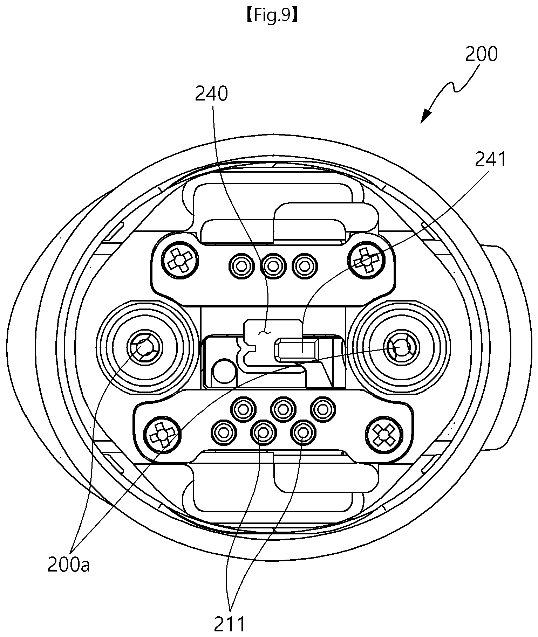

Meanwhile FIG. 8 is a plan view of an RF electrode cartridge for skin care and treatment in an embodiment of a handpiece for skin care and treatment according to the present disclosure and FIG. 9 is a bottom view of a handpiece body casing in an embodiment of a handpiece for skin care and treatment according to the present disclosure.

An embodiment of a handpiece for skin care and treatment according to the present disclosure is described hereafter in detail with reference to FIGS. 6 to 9.

In the cartridge insertion part 200b positioned at the front end of the handpiece body casing 200, the second body-connecting terminal part 211 to which the first body-connecting terminal part 192 is connected is positioned and the cartridge coupling block 210, in which a line connection part 212 connecting the first cooling water line part 170 and the second cooling water line part 180 to the cooling water circulation line part 200a is positioned, is movably positioned.

The electrode connection terminals 121a are electrically connected to the first body-connecting terminal part 192 through wires or cables and the second body-connecting terminal part 211 is electrically connected to the control body through a wire or a cable.

Accordingly, when the RF electrode cartridge for skin care and treatment according to the present disclosure is inserted in the cartridge insertion part 200b, the first body-connecting terminal part 192 is connected to the second body-connecting terminal part 211 of the cartridge coupling block 210, and the first cooling water line part 170 and the second cooling water line part 180 are connected to the cooling water circulation line part 200a while being inserted and fitted into the line connection part 212 of the cartridge coupling block 210.

Further, the cartridge coupling block 210 is positioned to be movable in the longitudinal direction of the handpiece body casing 200 and a spring member 220 for a cartridge that elastically supports the cartridge coupling block 210 is positioned in the handpiece body casing 200.

The cooling water circulation line part 200a may be a hose that has a sufficient length by at least the movement distance of the RF electrode cartridge 100 and can freely bend in the handpiece body casing 200 or may be a hose having a bellows structure of which the length can be adjusted in the handpiece body casing 200.

The spring member 220 for a cartridge elastically supports the cartridge coupling block 210 to which the RF electrode cartridge 100 is coupled and that is movable, thereby serving to reduce load that is applied to a patient when the RF electrode assembly 120 is pressed in close contact with the skin of the patient and pressing the RF electrode cartridge 100 to the skin of the patient with elastic restoring force such that the RF electrode assembly 120 is more completely brought in close contact with the skin of the patient.

A cartridge movement guide part 230 that guides straight movement of the cartridge coupling block 210 is positioned in the handpiece body casing 200.

The cartridge movement guide part 230 may include a movement guide rod part 231 positioned protrude from the cartridge coupling block 210 and a movement guide block 232 that is positioned in the handpiece body casing 200 and through which the movement guide rod part 231 is movably coupled.

Further, it is exemplified that a plurality of movement guide rod parts 231 is provided and at least any one of the plurality of movement guide rod parts is positioned through the spring member 220 for a cartridge that is a coil spring.

That is, it is exemplified that the spring member 220 for a cartridge is a coil spring, and a first end of the spring member 220 for a cartridge is supported in the cartridge insertion part 200b and a second end thereof is supported by the movement guide block 232 with the movement guide rod part 231 positioned through the spring member, whereby the spring member elastically supports straight movement of the cartridge coupling block 210 and the RF electrode cartridge 100 coupled to the cartridge coupling block 210.

Meanwhile, in the anti-burn RF electrode cartridge 100 for skin care and treatment according to the present disclosure, an electrode on-off switch 115 that turns on/off electrical power that is applied to the RF electrode assembly 120 is positioned on the top of the cartridge casing 110, that is, the top of the second casing part 200b.

The electrode on-off switch 115 includes a switch button 115a that is elastically pressed by a switch spring 115b, so it has a structure that turns on electrical power that is applied to the RF electrode assembly 120 only when the switch button 115a is pressed and that turns off the electrical power when the pressed switch button 115a is released.

Further, a switch-pressing protrusion 241 that presses the electrode on-off switch 115 when the cartridge coupling block 210 is pressed and moved is positioned to protrude in the handpiece body casing 200, that is, in the cartridge insertion part 200b.

The switch-pressing protrusion 241 is positioned to be in contact with the top of the switch button 115a with the RF electrode cartridge 100 coupled to the handpiece body casing 200.

In more detail, a switch-mounting protrusion 116 on the top of which the electrode on-off switch 115 is positioned is positioned on the top of the cartridge casing 110, that is, the top of the second casing part 200b, a protrusion insertion part 240 in which the switch-mounting protrusion 116 is inserted is positioned in the cartridge insertion part 200b, and the switch-pressing protrusion 241 is positioned to protrude in the protrusion insertion part 240 to face the switch-mounting protrusion 116.

The electrode on-off switch 115 is positioned such that the top of the switch button 115a protrudes from the top of the switch-mounting protrusion 116 or is level with the top of the switch protrusion, and when the RF electrode cartridge 100 is coupled to the handpiece body casing 200, the switch-mounting protrusion 116 is inserted in the protrusion insertion part 240 and the switch-pressing protrusion 241 is positioned to be in contact with the top of the switch button 115a.

That is, when a practitioner holds the handpiece body casing 200 and presses the handpiece body casing 200 with the RF electrode assembly 120 in contact with the skin, the RF electrode cartridge 100 is pushed and presses and moves the cartridge coupling block 210 while moving to the handpiece body casing 200, and accordingly, the switch-mounting protrusion 116 presses the switch button 115a, whereby electrical power is applied to the RF electrode assembly 120.

Further, when the handpiece body casing 200 is not pressed, that is, a practitioner does not presses the handpiece body casing 200 with the RF electrode assembly 120 in contact with the skin, the cartridge coupling block 210 is moved to the initial position by the spring member 220 for a cartridge and the switch button 115a is also returned to the initial position by the elastic restoring force of the switch spring 115b, so electrical power supplied to the RF electrode assembly 120 is cut, whereby the operation of the RF electrode assembly 120 is stopped.

According to the handpiece for skin care and treatment of the present disclosure, electrical power is applied to the RF electrode assembly 120 only when a practitioner holds and presses the handpiece body casing 200 during a procedure, and when the practitioner removes the force pressing the handpiece body casing 200, electrical power supplied to the RF electrode assembly 120 is automatically cut by the elastic restoring force of a spring, whereby it is possible to accurately perform a procedure on a necessary part an accurate number of times and it is also possible to prevent accidents such as injury to the skin due to excessive stimulation during treatment.

The anti-burn RF electrode cartridge 100 for skin care and treatment according to the present disclosure and the handpiece for skin care and treatment including the anti-burn RF electrode cartridge perform a procedure while moving the RF electrode assembly 120 in close contact with the skin of a patient.

In this case, the tip part 101 of the RF electrode cartridge 100 freely tilts within 360 degrees around the ball joint part 140 such that the RF electrode assembly 120, that is, the contact electrode part 121 can keep in close contact with the skin.

Further, vibration by the vibration motor part further improves the treatment effect of a procedure by generating a massage effect on the skin.

Further, the spring member 220 for a cartridge elastically attenuates the pressing force that is applied by a practitioner and the elastic restoring force of the spring member 220 for a cartridge can further keep the RF electrode assembly 120 in close contact with the skin.

Since the edge end of the RF electrode assembly that is the part where RF energy concentrates during a procedure is positioned to face up on the outer surface of the tip part 101 such that the part where RF energy concentrates during a procedure is spaced apart from the skin, the present disclosure prevents an accident of generation of a spark between the edge end of the RF electrode and the skin during a procedure, thereby having an effect of preventing a patient from getting a burn on the skin during skin treatment that uses an RF signal and greatly improving safety during a procedure.

Since the RF electrode assembly 120 moves in close contact with the skin while freely tilting within 360 degrees around the ball joint part 140 during a procedure, an accident such as injury to skin tissues or a burn on the skin when the RF electrode assembly 120 is separated from the skin is prevented, thereby greatly improving safety during a procedure.

## Claims

1. An anti-burn RF electrode cartridge (100) for skin care and treatment, the anti-burn RF electrode cartridge comprising:
a tip part (101) on a bottom of which an RF electrode assembly (120) configured to apply RF energy into skin is positioned; and
a cartridge casing (110) having an opening (110c) at a lower portion at which the tip part is positioned,
wherein the RF electrode assembly is positioned to cover the entire bottom of the tip part and extend to an outer surface of the tip part, thereby surrounding the outer surface of the tip part.

2. The anti-burn RF electrode cartridge of claim 1, wherein the RF electrode assembly comprises:
a contact electrode part covering the entire bottom of the tip part; and
a side electrode part connected to the contact electrode part and formed to surround a side of the tip part.

3. The anti-burn RF electrode cartridge of claim 2, wherein the contact electrode part and the side electrode part have an integrally connected structure, and an edge of the RF electrode assembly is positioned to face up on the outer surface of the tip part.

4. The anti-burn RF electrode cartridge of claim 1, wherein the RF electrode assembly is a plating layer integrally formed on the bottom and the outer surface of the tip part through plating.

5. The anti-burn RF electrode cartridge of claim 2, wherein the RF electrode assembly further comprises an insulating film member covering an entire surface of the contact electrode part and covering at least a portion of the side electrode part.

6. The anti-burn RF electrode cartridge of claim 2, wherein the RF electrode assembly is connected to an RF signal application wire part configured to apply AC power on the side electrode part.

7. The anti-burn RF electrode cartridge of claim 1, wherein the cartridge casing comprises:
a first casing part on a surface of which the RF electrode assembly is positioned;
a second casing part to which the first casing part is connected to be able to tilt and that has an opening at a lower portion through which the tip part passes;
a ball joint part configured to connect the second casing part and the tip part such that the first casing part can freely tilt within 360 degrees;
a bellows member of which a first end is connected to the first casing part and a second end is connected to the second casing part and that is positioned to surround the ball joint part and has a plurality of wrinkle parts; and
a bellows support frame member positioned at an upper end of the bellows member and formed in a frame structure surrounding an inner periphery of the bellows member.

8. The anti-burn RF electrode cartridge of claim 7, wherein the bellows support frame member has a plurality of frame fixing pins protruding on an outer surface thereof, and
a plurality of pin insertion parts in which the frame fixing pins are inserted is provided on the bellows member.

9. A handpiece for skin care and treatment, comprising:
an RF electrode cartridge comprising an RF electrode assembly supposed to come in contact with the skin; and
a handpiece body casing to which the RF electrode cartridge is separably coupled and that is connected to a control body,
wherein the RF electrode cartridge comprises the anti-burn RF electrode cartridge for skin care and treatment of any one of claims 1 to 8.

10. The handpiece for skin care and treatment of claim 9, wherein a cartridge insertion part in which the RF electrode cartridge is inserted and coupled is positioned at a front end of the handpiece body casing,
a cartridge coupling block configured to electrically connect the control body and the RF electrode assembly by being coupled with the RF electrode cartridge is movably positioned in the cartridge insertion part,
the cartridge coupling block is positioned in an elastically supported state by a spring member for a cartridge,
an electrode on-off switch configured to turn on and off electrical power that is applied to the RF electrode assembly is positioned on a top of the cartridge casing,
the electrode on-off switch comprises a switch button elastically supported by a switch spring and has a structure that turns on electrical power that is applied to the RF electrode assembly only when the switch button is pressed and that turns off electrical power when the pressed switch button is released, and
a switch-pressing protrusion configured to press the electrode on-off switch when the cartridge coupling block is pressed and moved inward is positioned to protrude in the cartridge insertion part.

11. The handpiece for skin care and treatment of claim 10, wherein a switch-mounting protrusion on a top of which the electrode on-off switch is positioned is positioned on a top of the cartridge casing and a protrusion insertion part in which the switch-mounting protrusion is inserted is positioned in the cartridge insertion part, and
the switch-pressing protrusion is positioned in the protrusion insertion part to face a top of the switch-mounting protrusion.

## Patentansprüche

1. Eine Verbrennungsschutz-HF-Elektrodenkartusche zur Hautpflege und -behandlung, wobei die Verbrennungsschutz-HF-Elektrodenkartusche folgende Merkmale aufweist:
ein Spitzenteil, an dessen Boden eine HF-Elektrodenanordnung positioniert ist, die dazu konfiguriert ist, HF-Energie in die Haut einzubringen; und
ein Kartuschengehäuse mit einer Öffnung an einem unteren Abschnitt, an dem das Spitzenteil positioniert ist,
wobei die HF-Elektrodenanordnung dahingehend positioniert ist, den gesamten Boden des Spitzenteils zu bedecken und sich zu einer Außenfläche des Spitzenteils zu erstrecken, wodurch dieselbe die Außenfläche des Spitzenteils umgibt.

2. Die Verbrennungsschutz-HF-Elektrodenkartusche gemäß Anspruch 1, wobei die HF-Elektrodenanordnung folgende Merkmale aufweist:
ein Kontaktelektrodenteil, das den gesamten Boden des Spitzenteils bedeckt; und
ein Seitenelektrodenteil, das mit dem Kontaktelektrodenteil verbunden und dahingehend ausgebildet ist, eine Seite des Spitzenteils zu umgeben.

3. Die Verbrennungsschutz-HF-Elektrodenkartusche gemäß Anspruch 2, wobei das Kontaktelektrodenteil und das Seitenelektrodenteil eine einstückig verbundene Struktur aufweisen und eine Kante der HF-Elektrodenanordnung dahingehend positioniert ist, der Außenfläche des Spitzenteils zugewandt zu sein.

4. Die Verbrennungsschutz-HF-Elektrodenkartusche gemäß Anspruch 1, wobei die HF-Elektrodenanordnung eine Plattierungsschicht ist, die durch Plattieren einstückig auf dem Boden und der Außenfläche des Spitzenteils ausgebildet ist.

5. Die Verbrennungsschutz-HF-Elektrodenkartusche gemäß Anspruch 2, wobei die HF-Elektrodenanordnung ferner ein Isolierfilmelement aufweist, das eine gesamte Oberfläche des Kontaktelektrodenteils bedeckt und zumindest einen Abschnitt des Seitenelektrodenteils bedeckt.

6. Die Verbrennungsschutz-HF-Elektrodenkartusche gemäß Anspruch 2, wobei die HF-Elektrodenanordnung mit einem HF-Signalaufbringungsdrahtteil verbunden ist, der dazu konfiguriert ist, Wechselstrom an das Seitenelektrodenteil anzulegen.

7. Die Verbrennungsschutz-HF-Elektrodenkartusche gemäß Anspruch 1, wobei das Kartuschengehäuse folgende Merkmale aufweist:
ein erstes Gehäuseteil, an dessen Oberfläche die HF-Elektrodenanordnung positioniert ist;
ein zweites Gehäuseteil, mit dem das erste Gehäuseteil verbunden ist, um kippen zu können, und das eine Öffnung an einem unteren Abschnitt aufweist, durch die das Spitzenteil verläuft;
ein Kugelgelenkteil, das dazu konfiguriert ist, das zweite Gehäuseteil und das Spitzenteil derart zu verbinden, dass das erste Gehäuseteil innerhalb von 360 Grad frei kippen kann;
ein Balgelement, dessen erstes Ende mit dem ersten Gehäuseteil verbunden ist und dessen zweites Ende mit dem zweiten Gehäuseteil verbunden ist und das dahingehend positioniert ist, das Kugelgelenkteil zu umgeben, und das eine Mehrzahl von Faltenteilen aufweist; und
ein Balgstützrahmenelement, das an einem oberen Ende des Balgelements positioniert und in einer Rahmenstruktur ausgebildet ist, die einen Innenumfang des Balgelements umgibt.

8. Die Verbrennungsschutz-HF-Elektrodenkartusche gemäß Anspruch 7, wobei das Balgstützrahmenelement eine Mehrzahl von Rahmenbefestigungsstiften aufweist, die an einer Außenfläche davon vorstehen, und
eine Mehrzahl von Stifteinsetzteilen, in die die Rahmenbefestigungsstifte eingesetzt sind, an dem Balgelement bereitgestellt ist.

9. Ein Handstück zur Hautpflege und -behandlung, das folgende Merkmale aufweist:
eine HF-Elektrodenkartusche, die eine HF-Elektrodenanordnung aufweist, die mit der Haut in Kontakt kommen soll; und
ein Handstückkörpergehäuse, mit dem die HF-Elektrodenkartusche trennbar gekoppelt ist und das mit einem Steuerkörper verbunden ist,
wobei die HF-Elektrodenkartusche die Verbrennungsschutz-HF-Elektrodenkartusche zur Hautpflege und -behandlung gemäß einem der Ansprüche 1 bis 8 aufweist.

10. Das Handstück zur Hautpflege und -behandlung gemäß Anspruch 9, wobei ein Kartuscheneinsetzteil, in das die HF-Elektrodenkartusche eingesetzt und gekoppelt ist, an einem vorderen Ende des Handstückkörpergehäuses positioniert ist,
ein Kartuschenkopplungsblock, der dazu konfiguriert ist, den Steuerkörper und die HF-Elektrodenanordnung elektrisch zu verbinden, indem er mit der HF-Elektrodenkartusche gekoppelt ist, beweglich in dem Kartuscheneinsetzteil positioniert ist,
der Kartuschenkopplungsblock in einem elastisch gestützten Zustand durch ein Federelement für eine Kartusche positioniert ist,
ein Elektroden-Ein-Aus-Schalter, der dazu konfiguriert ist, elektrische Leistung, die an die HF-Elektrodenanordnung angelegt wird, ein- und auszuschalten, an einer Oberseite des Kartuschengehäuses positioniert ist,
der Elektroden-Ein-Aus-Schalter einen Schaltknopf aufweist, der elastisch durch eine Schaltfeder gestützt ist und eine Struktur aufweist, die elektrische Leistung, die an die HF-Elektrodenanordnung angelegt wird, nur einschaltet, wenn der Schaltknopf gedrückt wird, und die elektrische Leistung ausschaltet, wenn der gedrückte Schaltknopf freigegeben wird, und
ein Schalterdrückvorsprung, der dazu konfiguriert ist, den Elektroden-Ein-Aus-Schalter zu drücken, wenn der Kartuschenkopplungsblock gedrückt und nach innen bewegt wird, positioniert ist, um in dem Kartuscheneinsetzteil vorzustehen.

11. Das Handstück zur Hautpflege und -behandlung gemäß Anspruch 10, wobei ein Schaltermontagevorsprung, an dessen Oberseite der Elektroden-Ein-Aus-Schalter positioniert ist, an einer Oberseite des Kartuschengehäuses positioniert ist und ein Vorsprungeinsetzteil, in das der Schaltermontagevorsprung eingesetzt ist, in dem Kartuscheneinsetzteil positioniert ist, und
der Schalterdrückvorsprung in dem Vorsprungeinsetzteil positioniert ist, um einer Oberseite des Schaltermontagevorsprungs zugewandt zu sein.

## Revendications

1. Cartouche d'électrode RF anti-brûlure (100) pour les soins et le traitement de la peau, la cartouche d'électrode RF anti-brûlure comprenant :
une partie d'embout (101) sur une partie inférieure de laquelle est positionné un ensemble d'électrodes RF (120) configuré pour appliquer de l'énergie RF dans la peau ; et
un boîtier de cartouche (100) comportant une ouverture (100c) dans une partie inférieure dans laquelle est positionnée la partie d'extrémité,
dans laquelle l'ensemble d'électrodes RF est positionné pour recouvrir l'ensemble de la partie inférieure de la partie de pointe et à se prolonger jusqu'à une surface extérieure de la partie de pointe, entourant ainsi la surface extérieure de la partie de pointe.

2. Cartouche d'électrode RF anti-brûlure selon la revendication 1, dans laquelle l'ensemble d'électrodes RF comprend :
une partie d'électrode de contact recouvrant l'ensemble de la partie inférieure de la partie de pointe ; et
une partie d'électrode latérale connectée à la partie d'électrode de contact et formée pour entourer un côté de la partie de pointe.

3. Cartouche d'électrode RF anti-brûlure selon la revendication 2, dans laquelle la partie d'électrode de contact et la partie d'électrode latérale ont une structure connectée de manière intégrale, et un bord de l'ensemble d'électrodes RF est positionné pour être orienté vers le haut sur la surface extérieure de la partie de pointe.

4. Cartouche d'électrode RF anti-brûlure selon la revendication 1, dans laquelle l'ensemble d'électrodes RF est une couche de placage formée d'un seul tenant sur la partie inférieure et la surface extérieure de la partie de pointe par placage.

5. Cartouche d'électrode RF anti-brûlure selon la revendication 2, dans laquelle l'ensemble d'électrodes RF comprend en outre un élément de film isolant recouvrant une surface intégrale de la partie d'électrode de contact et recouvrant au moins une partie de la partie d'électrode latérale.

6. Cartouche d'électrode RF anti-brûlure selon la revendication 2, dans laquelle l'ensemble d'électrodes RF est connecté à une partie de fil d'application de signal RF configurée pour appliquer une alimentation CA sur la partie d'électrode latérale.

7. Cartouche d'électrode RF anti-brûlure selon la revendication 1, dans laquelle le boîtier de la cartouche comprend :
une première partie de boîtier sur une surface de laquelle est positionné l'ensemble d'électrodes RF ;
une deuxième partie de boîtier à laquelle la première partie de boîtier est connectée de manière à pouvoir s'incliner et qui comporte une ouverture dans une partie inférieure à travers laquelle passe la partie de pointe ;
une partie de pièce à rotule configurée pour connecter la deuxième partie de boîtier et la partie de pointe de telle sorte que la première partie de boîtier puisse pivoter librement à 360 degrés ;
un élément à soufflet dont une première extrémité est connectée à la première partie de boîtier et une seconde extrémité est connectée à la seconde partie de boîtier, qui est positionnée pour entourer la partie de pièce à rotule et qui comporte une pluralité de parties plissées ; et
un élément de support du soufflet positionné à une extrémité supérieure de l'élément de soufflet et formé dans une structure de cadre entourant une périphérie intérieure de l'élément de soufflet.

8. Cartouche d'électrode RF anti-brûlure selon la revendication 7, dans laquelle l'élément de support à soufflet comporte une pluralité de broches de fixation de cadre faisant saillie sur sa surface extérieure, et
une pluralité de parties d'insertion de broches dans lesquelles les broches de fixation du cadre sont insérées est prévue sur l'élément à soufflet.

9. Pièce à main pour le soin et le traitement de la peau, comprenant :
une cartouche d'électrode RF comprenant un ensemble d'électrodes RF pour entrer en contact avec la peau ; et
un boîtier de corps de pièce à main auquel la cartouche d'électrode RF est couplée de manière amovible et qui est relié à un corps de commande,
dans lequel la cartouche d'électrode RF comprend la cartouche d'électrode RF anti-brûlure pour les soins de la peau et le traitement selon l'une quelconque des revendications 1 à 8.

10. Pièce à main pour les soins et le traitement de la peau selon la revendication 9, dans laquelle une partie d'insertion de cartouche dans laquelle la cartouche d'électrode RF est insérée et accouplée est positionnée à une extrémité avant du boîtier de corps de pièce à main,
un bloc d'accouplement de cartouche configuré pour connecter électriquement le corps de commande et l'ensemble d'électrodes RF en étant accouplé à la cartouche d'électrodes RF est positionné de manière mobile dans la partie d'insertion de cartouche,
le bloc d'accouplement de cartouche est positionné dans un état supporté de manière élastique par un élément ressort pour une cartouche,
un interrupteur marche-arrêt d'électrode configuré pour activer et désactiver l'alimentation électrique qui est appliquée à l'ensemble d'électrodes RF est positionné sur le dessus du boîtier de la cartouche,
l'interrupteur marche-arrêt de l'électrode comprend un bouton d'interrupteur soutenu de manière élastique par un ressort d'interrupteur et présente une structure qui active l'alimentation électrique appliquée à l'ensemble d'électrodes RF uniquement lorsque le bouton d'interrupteur est enfoncé et qui désactive l'alimentation électrique lorsque le bouton d'interrupteur enfoncé est relâché, et
une saillie d'enfoncement de l'interrupteur, configurée pour actionner l'interrupteur marche-arrêt de l'électrode lorsque le bloc d'accouplement de la cartouche est enfoncé et déplacé vers l'intérieur, est positionnée de manière à faire saillie dans la partie d'insertion de la cartouche.

11. Pièce à main pour les soins et le traitement de la peau selon la revendication 10, dans laquelle une saillie de montage d'interrupteur sur le dessus de laquelle est positionné l'interrupteur marche-arrêt de l'électrode est positionnée sur le dessus du boîtier de la cartouche et une partie d'insertion de saillie dans laquelle la saillie de montage d'interrupteur est insérée est positionnée dans la partie d'insertion de la cartouche, et
la saillie d'enfoncement de l'interrupteur est positionnée dans la partie d'insertion de la saillie pour faire face à une partie supérieure de la saillie de montage d'interrupteur.
